# EUROPÄISCHE PATENTSCHRIFT

(11) **EP 0 785 152 B1**
(45) Veröffentlichungstag und Bekanntmachung des Hinweises auf die Patenterteilung: **14.03.2001**
(21) Anmeldenummer: 96120653.9
(22) Anmeldetag: 20.12.1996
(51) Int. Cl.: B65F 1/14, F16B 47/00, A61B 19/02

(54) **Standfuss, insbesondere für einen Abfallbehälter für medizinische Abfälle**
Stand, particularly for a medical waste receptacle
Support, notamment pour récipient à déchets médicaux

(30) Priorität: 17.01.1996 DE 19601552
(43) Veröffentlichungstag der Anmeldung: 23.07.1997
(73) Patentinhaber: Udo Heisig GmbH, 85609 Aschheim-Dornach (DE)
(72) Erfinder: Heisig, Udo, 85609 Ashheim-Dornach (DE)
(74) Vertreter: Feldkamp, Rainer, Dipl.-Ing.

(56) Entgegenhaltungen:
- DE-U- 9 112 440
- DE-U- 29 517 460
- US-A- 5 076 527
- US-A- 5 176 357

## Beschreibung

Die Erfindung bezieht sich auf einen Standfuß, insbesondere für einen Abfallbehälter für medizinische Abfälle wie z.B. Injektionsspritzen, der im Oberbegriff des Patentanspruchs 1 genannten Art.

Bei der medizinischen Versorgung fallen häufig Abfälle wie Injektionsspritzen an, die in speziellen Abfallbehältern entsorgt werden müssen, um auszuschließen, daß sich Personen, die mit diesen Abfällen in Berührung kommen, verletzen oder infizieren.

Zu diesem Zweck wurde in der DE-U-295 17 460 bereits vorgeschlagen, einen Standfuß mit einem Standsockel zu verwenden, unter dessen Standfläche ein elastisch biegsames, plattenförmiges Element angeordnet ist, das sich auf der Abstellfläche, auf der der Standfuß bzw. der Abfallbehälter abgestellt wird, festsaugt. Der Abfallbehälter ist somit gegen ein Umkippen geschützt, weswegen er bei Gebrauch, d.h. wenn zum Beispiel benutzte Injektionsspritzen eingeführt werden, nicht festgehalten zu werden braucht. Hierdurch wird das Verletzungs- bzw. Infektionsrisiko für das medizinische Personal erheblich reduziert.

Es ist zu beachten, daß diese Abfallbehälter im wesentlichen für den Einmal-Gebrauch vorgesehen sind, weswegen eine kostengünstige Herstellung erforderlich ist.

Da der durch das elastisch biegsame, plattenförmige Element verursachte Saugeffekt sehr stark sein kann, besteht bei Abfallbehältern mit derartigem Standfuß die Gefahr, daß der Behälter nach dem Gebrauch nur sehr schwer wieder von dem Gegenstand, auf dem er abgestellt wurde, gelöst werden kann. Insbesondere dann, wenn der Behälter nicht parallel zur Oberfläche des Gegenstandes weggezogen werden kann, muß die Saugwirkung durch ruckartiges Anheben des Behälters aufgehoben werden, wobei die medizinischen Abfälle aus dem Behälter geschleudert werden können.

Der Erfindung liegt die Aufgabe zugrunde, einen Standfuß der eingangs genannten Art zu schaffen, der leicht und kostengünstig hergestellt werden kann, und der trotz guter Saugwirkung problemlos wieder von der Abstellfläche gelöst werden kann.

Diese Aufgabe wird durch die in Anspruch 1 angegebenen Merkmale gelöst.

Vorteilhafte Ausgestaltungen und Weiterbildungen der Erfindung ergeben sich aus den Unteransprüchen.

Nachdem der medizinische Abfall in den Abfallbehälter mit dem erfindungsgemäßen Standfuß eingeführt wurde, läßt sich der Behälter problemlos von der Abstellfläche lösen, indem die Lösevorrichtung betätigt wird, wodurch der Saugeffekt umgehend aufgehoben wird.

Ausführungsbeispiele der Erfindung werden im folgenden anhand der Zeichnung noch näher erläutert.

In der Zeichnung zeigen:
Fig. 1 eine perspektivische Ansicht eines Abfallbehälters mit einer ersten Ausführungsform des Standfußes,
Fig. 2 eine schematische Schnittansicht entlang der Linie I-I des Abfallbehälters bzw. Standfußes nach Fig. 1,
Fig. 3 eine perspektivische Ansicht eines Abfallbehälters mit einer zweiten Ausführungsform des Standfußes,
Fig. 4 eine schematische Schnittansicht entlang der Linie II-II des Abfallbehälters bzw. Standfußes nach Fig. 3.

Im folgenden wird der Standfuß in einer spezielle Anwendung als Standfuß eines Abfallbehälters näher erläutert, obwohl die Anwendung des Standfußes selbstverständlich nicht auf diese Anwendung beschränkt ist.

Bei den gezeigten Ausführungsformen ist eine Abfallbox 1 mit dem Standfuß ausgestattet. Der Standfuß weist einen Standsockel 2 auf, dessen untenliegende Standfläche mit 3 bezeichnet ist. Der Standfuß ist durch eine form- und/oder kraftschlüssige, lösbare Verbindung mit der Abfallbox 1 verbunden. Die form- und/oder kraftschlüssige Verbindung kann dabei beispielsweise durch einen Bajonettverschluß gebildet sein.

Obwohl dies nicht gezeigt ist, ist es selbstverständlich auch denkbar, die Abfallbox 1 und den Standfuß einstückig auszubilden.

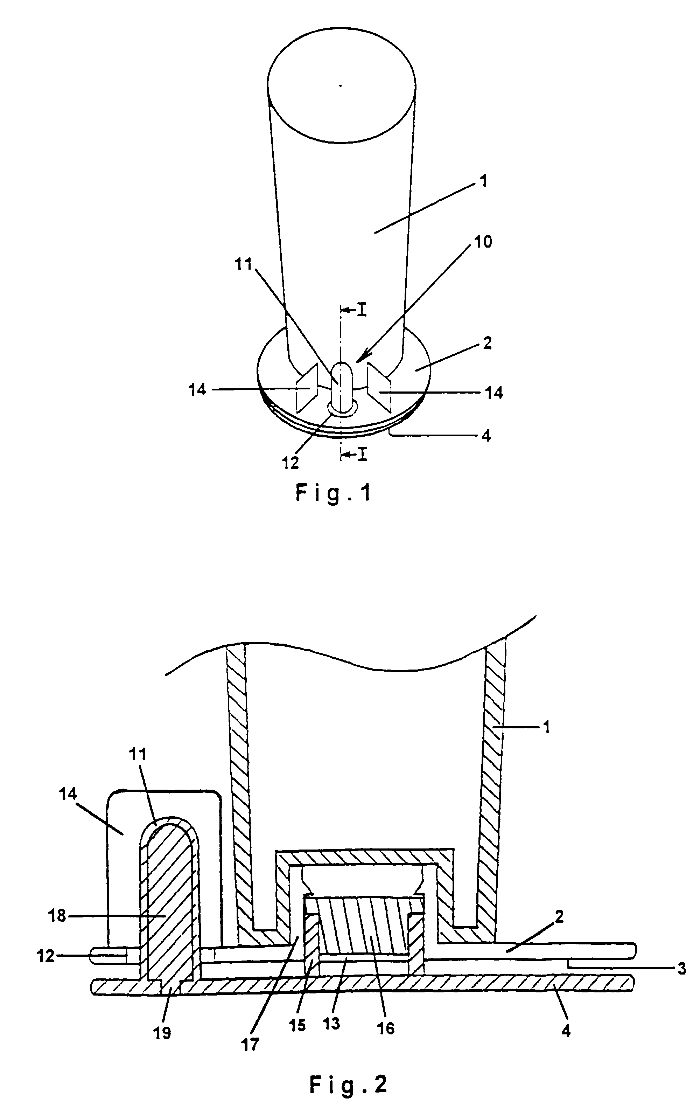

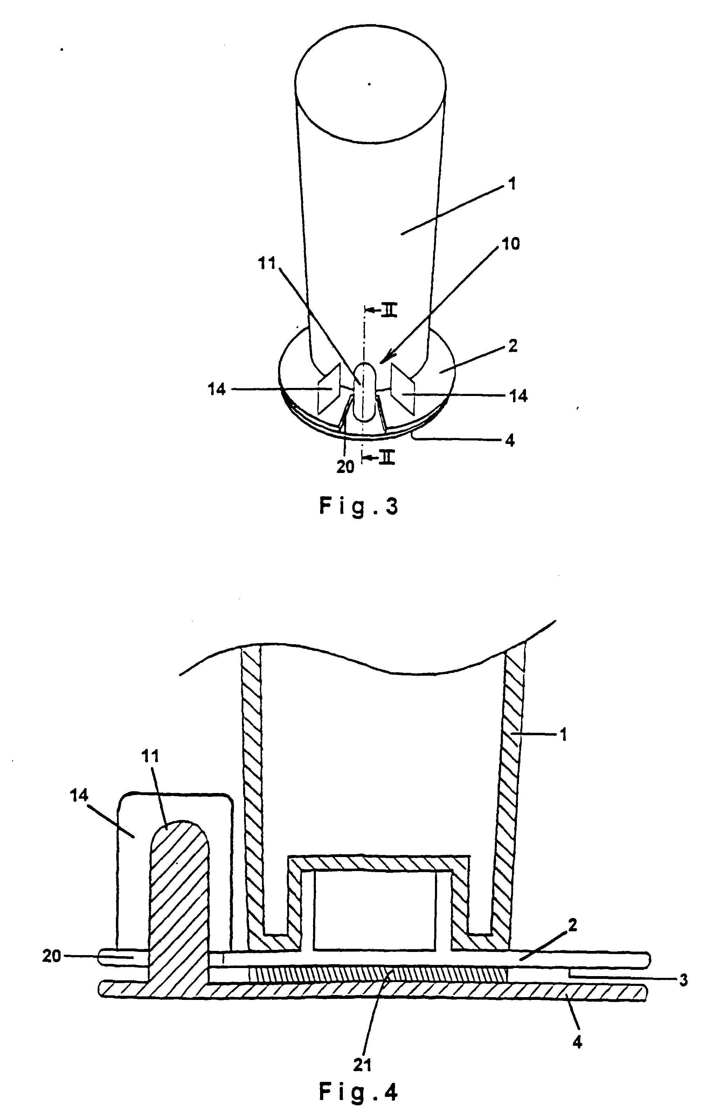

Ein elastisch biegsames, plattenförmiges Element 4 ist an der Standfläche 3 an deren Mittelbereich befestigt, wobei die Fläche des elastisch biegsamen plattenförmigen Elementes 4 im wesentlichen durch die Standfläche 3 abgedeckt ist. Der Abstand zwischen der Standfläche 3 und dem elastisch biegsamen plattenförmigen Material 4, ist in Figur 2 und 4 aus Übersichtlichkeitsgründen übertrieben groß dargestellt. In der Praxis liegt das elastisch biegsame plattenförmige Element 4 an der Standfläche 3 an.

Gemäß der Ausführungsform nach Figur 1 ist die insgesamt mit 10 bezeichnete Lösevorrichtung durch ein Element 11 gebildet, das an der Oberseite des elastisch-biegsamen, plattenförmigen Elementes 4 derart angeordnet ist, daß es sich zumindest teilweise durch einen Durchbruch 12 im Standsockel 2 erstreckt. Der Durchbruch 12 ist dabei so gebildet, daß sich das Element 11 nicht verkeilt bzw. verklemmt, wenn es mit den Fingern leicht gekippt oder angehoben wird.

Gemäß der Ausführungsform nach Figur 3 erstreckt sich das Element 11 zumindest teilweise durch eine nach außen offene Aussparung 20, die im Randbereich des Standsockels 2 angeordnet ist.

Das Element 11 kann entweder wie gezeigt einstückig mit dem elastisch biegsamen plattenförmigen Element 4 hergestellt oder später auf der von der Abstellfläche abgewandten Oberfläche des elastisch biegsamen plattenförmigen Elementes 4 befestigt werden.

Gemäß der Ausführungsform nach Figur 1 kann das Element 11 aus elastisch biegsamen Material derart gebildet sein, daß sich ein hohler Innenraum 18 ergibt, der mit einem Kanal bzw. Durchbruch 19 im elastisch biegsamen plattenförmigen Element in Verbindung steht, so daß die Luft, die sich in dem hohlen Element 11 befindet, durch den Kanal 19 strömt, wenn das Element 11 zusammengepreßt wird. Die durch den Kanal 19 strömende Luft erleichtert die Aufhebung des Saugeffektes.

Es ist ebenfalls denkbar, das Element 11 aus elastisch biegsamen Material massiv auszubilden, wie dies in Figur 3 gezeigt ist. In diesem Fall führt, ebenso wie im erstgenannten Fall, bereits ein Kippen oder Anheben des Elementes 11 zu einer Aufhebung des Saugeffektes.

Die Befestigung des elastisch biegsamen plattenförmigen Elementes 4 am Mittelbereich der Standfläche 3 des Standsockels 2 kann auf verschiedene Arten erfolgen. Denkbar sind beispielsweise einfache Klebe-Verbindungen, Einrast-Verbindungen oder Klemm-Verbindungen.

Bei der Ausführungsform nach Figur 1 ist eine Klemm-Verbindung vorgesehen. Das elastisch biegsame plattenförmige Element 4 weist dazu im Mittelbereich seiner Oberfläche einen vorzugsweise ringförmigen Steg 15 auf, der sich zumindest teilweise durch einen Durchbruch 13 erstreckt, der derart im Standsockel 2 angeordnet ist, daß er mit dem ringförmigen Steg 15 ausgerichtet ist. Der Standsockel 2 weist an seiner Oberseite weiterhin einen ebenfalls vorzugsweise ringförmigen Steg 17 auf, der am Rand des Durchbruchs 13 angeordnet ist und Bestandteil der Verbindung zur Abfallbox 1 sein kann. Der ringförmige Steg 15 liegt mit seinem Außenumfang am Innenumfang des Steges 17 an. Ein Verriegelungselement 16 ist von oben und unter Spannung derart in den ringförmigen Steg 15 eingeführt, daß dessen Außenumfang an den Innenumfang des Steges 17 gepreßt wird, wodurch eine feste Verbindung des elastisch biegsamen plattenförmigen Elementes 4 mit dem Standsockel 2 gebildet ist.

Bei der Ausführungsform nach Figur 3 ist das elastisch biegsame plattenförmige Element 4 durch eine Klebe-Verbindung an dem Standsockel 2 befestigt. Der in Figur 4 zur Verdeutlichung übertrieben stark dargestellte Klebebereich 21 kann wie gezeigt, hinsichtlich seiner Abmessungen in der Größenordnung der Bodenfläche der Abfallbox 1 ausgebildet sein, so daß ein freier Umfangsrand verbleibt. Es kann jede geeignete Art von Kleber verwendet werden; auch ein beidseitiges Klebeband ist denkbar.

Wie bereits erwähnt, kann die Verbindung des Standfußes mit der Abfallbox 1 durch einen Bajonettverschluß oder irgendeinen anderen Verschluß, der durch das Gegeneinanderdrehen des Standsockels 2 gegen die Abfallbox 1 geschlossen bzw. gelöst wird, erfolgen. Um das Lösen bzw. Schließen dieses Verschlusses zu erleichtern, weist der Standsockel 2 im Außenbereich seiner Oberseite, also dem Bereich, der nicht von der Abfallbox 1 verdeckt wird, eine Halte- bzw. Griffvorrichtung auf. Vorzugsweise ist diese Halte- bzw. Griffvorrichtung durch Stege 14 gebildet, die im wesentlichen radial und senkrecht zur Oberfläche des Standsockels 2 angeordnet sind. Das medizinische Personal kann die Abfallbox 1 vom Standsockel 2 lösen, indem es die Stege 14 des Standsockels 2 zwischen Zeigefinger und Daumen der einen Hand greift, wodurch der Standsockel 2 gegen ein Verdrehen geschützt ist, während es die Abfallbox 1 mit der anderen Hand dreht, um sie zu lösen.

Wenn zwei Stege 14, wie gezeigt, seitlich von der Lösevorrichtung 10 angeordnet sind, ist die Lösevorrichtung 10 gegen eine versehentliche Betätigung geschützt.

## Patentansprüche

1. Standfuß, insbesondere für einen Abfallbehälter für medizinische Abfälle wie zum Beispiel Injektionsspritzen mit einem Standsockel (2), der eine Standfläche (3) aufweist, unter der ein elastisch biegsames plattenförmiges Element (4) angeordnet ist, das in seinem Mittelbereich mit dem Standsockel (2) verbunden ist,
dadurch gekennzeichnet, daß der Standfuß eine Lösevorrichtung (10) aufweist, die durch ein Element (11) gebildet ist, das auf der Oberseite des elastisch biegsamen plattenförmigen Elementes (4) angeordnet ist und sich zumindest teilweise durch eine Aussparung (12;20) im Randbereich des Standsockels (2) erstreckt, wobei die Aussparung (12;20) so bemessen ist, daß sich das Element (11) nicht verkeilt bzw. verklemmt.

2. Standfuß nach Anspruch 1, dadurch gekennzeichnet,
daß das Element (11) aus elastisch biegsamen Material derart gebildet ist, daß sich ein hohler Innenraum (18) ergibt, der mit einem Kanal (19) im elastisch biegsamen plattenförmigen Element (4) in Verbindung steht, so daß die Luft, die sich in dem Element (11) befindet, durch den Kanal strömt, wenn das Element (11) zusammengepreßt wird.

3. Standfuß nach Anspruch 1, dadurch gekennzeichnet,
daß das Element (11) aus elastisch biegsamen Material und einstückig mit dem elastisch biegsamen plattenförmigen Element (4) gebildet ist.

4. Standfuß nach Anspruch 1 bis 3, dadurch gekennzeichnet,
daß das elastisch biegsame Element (4) im Mittelbereich seiner Oberseite mindestens einen im wesentlichen ringförmigen Steg (15) aufweist, der in einen Durchbruch (13) im Standsockel (2) einsteckbar ist, und daß das elastisch biegsame Element (4) mit dem Sockel (2) unter Mitwirkung des Steges (15) durch ein Verriegelungselement verbunden ist.

5. Standfuß nach Anspruch 4, dadurch gekennzeichnet,
daß das Verriegelungselement durch einen Stopfen (16) gebildet ist, der beim Einstecken in den Steg (15) diesen auseinanderspreizt.

6. Standfuß nach Anspruch 1 bis 3, dadurch gekennzeichnet,
daß das elastisch biegsame plattenförmige Element (4) durch eine Klebe-Verbindung an der Standfläche (3) des Standsockels (2) befestigt ist.

7. Standfuß nach Anspruch 6, dadurch gekennzeichnet,
daß der Klebebereich (21) in der Größenordnung der Grundfläche des von dem Standfuß getragenen Gegenstandes (1) gebildet ist.

8. Standfuß nach Anspruch 1 bis 7, dadurch gekennzeichnet,
daß ein Behälter (1) durch eine kraft- und/oder formschlüssige Verbindung mit dem Standfuß lösbar verbunden ist, daß diese Verbindung durch ein gegeneinander Verdrehen von Behälter (1) und Standfuß hervorgerufen bzw. gelöst wird, und daß der Standsockel (2) und/oder die Entsorgungsbox (1) eine Halte- bzw. Griffvorrichtung (14) aufweist, die das gegeneinander Verdrehen erleichtert.

9. Standfuß nach Anspruch 8, dadurch gekennzeichnet,
daß die Halte- bzw. Griffvorrichtung des Standsockels (2) durch mindestens einen Steg (14) gebildet ist, der auf der Oberseite des Standsockels (2) angeordnet ist.

10. Standfuß nach Anspruch 9, dadurch gekennzeichnet,
daß der oder die Stege (14) bezüglich des Standsockels (2) im wesentlichen radial angeordnet sind.

11. Standfuß nach Anspruch 10, dadurch gekennzeichnet,
daß zwei Stege (14) benachbart zur Lösevorrichtung (10) und auf unterschiedlichen Seiten derart angeordnet sind, daß die Lösevorrichtung (10) nicht versehentlich betätigt werden kann.

## Claims

1. Stabilising foot, more particularly for a refuse container for medical waste such as for example hypodermic syringes, having a stabilising pedestal (2) which incorporates a stabilising surface (3) beneath which there is arranged an elastically flexible plate-shaped element (4) which in its central zone is joined to the stabilising pedestal (2),
characterised in that the stabilising foot incorporates a releasing device (10) constituted by an element (11) which is arranged on the upper face of the elastically flexible plate-shaped element (4) and extends at least partially through a recess (12; 20) in the peripheral zone of the stabilising pedestal (2), the recess (12; 20) having dimensions such that the element (11) does not become wedged tight or jammed.

2. Stabilising foot according to claim 1, characterised in that the element (11) made of elastically flexible material is constituted in such a manner as to give rise to an internal cavity (18) which communicates with a duct (19) in the elastically flexible plate-shaped element (4), with the result that the air in the element (11) flows through the duct when the element (11) is compressed.

3. Stabilising foot according to claim 1, characterised in that the element (11) is formed from elastically flexible material integrally with the elastically flexible plate-shaped element (4).

4. Stabilising foot according to claims 1 to 3, characterised in that in the central zone of its upper face the elastically flexible element (4) incorporates at least one substantially annular land (15) which is adapted to be inserted into a hole (13) in the stabilising pedestal (2), and that with the assistance of the land (15) the elastically flexible element (4) is joined to the pedestal (2) by a locking element.

5. Stabilising foot according to claim 4, characterised in that the locking element is constituted by a stopper (16) which splays the land (15) apart upon being inserted thereinto.

6. Stabilising foot according to claims 1 to 3, characterised in that the elastically flexible plate-shaped element (4) is fixed to the stabilising surface (3) of the stabilising pedestal (2) by an adhesive-bonded joint.

7. Stabilising foot according to claim 6, characterised in that the adhesive bonding zone (21) is roughly the same size as the bottom surface of the object (1) being supported by the stabilising foot.

8. Stabilising foot according to claims 1 to 7, characterised in that a container (1) is releasably joined to the stabilising foot by a frictional and/or interlocking connection, that this connection is instituted and released by twisting the container (1) and the stabilising foot against one another, and that the stabilising pedestal (2) and/or the waste bin (1) incorporates a holding or gripping device (14) which facilitates said twisting action.

9. Stabilising foot according to claim 8, characterised in that the holding or gripping device of the stabilising pedestal (2) is constituted by at least one land (14) which is disposed on the upper face of the stabilising pedestal (2).

10. Stabilising foot according to claim 9, characterised in that the land(s) (14) is/are disposed substantially radially in relation to the stabilising pedestal (2).

11. Stabilising foot according to claim 10, characterised in that two lands (14) are disposed adjoining the releasing device (10) and on different sides, in such a manner that the release device (10) cannot be operated inadvertently.

## Revendications

1. Support, en particulier support pour un récipient à déchets médicaux, tels que par exemple des seringues d'injection, comprenant un socle de support (2) avec une surface d'appui (3) sous laquelle est disposé un élément (4) en forme de plaque déformable élastiquement qui est lié au socle de support (2) dans sa partie médiane, caractérisé en ce que le socle de support comporte un dispositif de séparation (10) qui est formé d'un élément (11) disposé sur la face supérieure de l'élément (4) en forme de plaque, déformable élastiquement, et s'étend au moins partiellement à travers un évidement (12; 20) dans la région du bord du socle de support (2), l'évidement (12; 20) étant dimensionné de manière telle que l'élément (11) ne puisse pas se coincer .

2. Support selon la revendication 1, caractérisé en ce que l'élément (11) est réalisé en matériau déformable élastiquement de manière à former une chambre intérieure (18) creuse qui communique avec un canal (19) dans l'élément (4) en forme de plaque, déformable élastiquement, de telle sorte que l'air qui est présent dans l'élément (11) s'écoule à travers le canal lorsqu'on presse l'élément (11).

3. Support selon la revendication 1, caractérisé en ce que l'élément (11) est réalisé en matériau déformable élastiquement et est formé d'une pièce avec l'élément (4) en forme de plaque, déformable élastiquement.

4. Support selon les revendications 1 à 3, caractérisé en ce que l'élément (4) en forme de plaque, déformable élastiquement, dans la région médiane de sa surface supérieure comporte au moins une cloison (15) essentiellement annulaire qui peut s'engager dans un passage (13) dans le socle support (2) et en ce que l'élément (4) en forme de plaque, déformable élastiquement, est lié au socle de support (2) - avec la participation de la cloison (15) - par un élément de verrouillage.

5. Support selon la revendication 4, caractérisé en ce que l'élément de verrouillage est formé d'un bouchon (16) qui lorsqu'il s'engage dans le rebord (15) écarte celui)-ci.

6. Support selon les revendications 1 à 3, caractérisé en ce que l'élément (4) en forme de plaque, déformable élastiquement, est fixé à la surface d'appui (3) du socle de support (2) par une liaison collée.

7. Support selon la revendication 6, caractérisé en ce que la zone de collage (21) est du même ordre de grandeur que la surface de base de l'objet (1) supporté par le support.

8. Support selon les revendications 1 à 7, caractérisé en ce qu'un récipient (1) est lié de manière séparable au support par une liaison par serrage et/ou par complémentarité de formes, en ce que cette liaison peut être mise en oeuvre ou libérée par rotation du support et du récipient (1) l'un par rapport à l'autre et en ce que le socle de support (2) et/ou la boîte à déchets (1) présente un dispositif de préhension ou un dispositif formant poignée (14) qui facilite la rotation.

9. Support selon la revendication 8, caractérisé en ce que le dispositif de préhension ou de poignée du socle de support (2) est formé d'au moins une cloison (14) qui est disposée sur la face supérieure du support (2).

10. Support selon la revendication 9, caractérisé en ce que la ou les cloison(s) (14) est (sont) disposée(s) essentiellement radialement par rapport au socle de support.

11. Support selon la revendication 10, caractérisé en ce que deux cloisons (14) sont disposées dans le voisinage du dispositif de déverrouillage (10), sur des côtés différents, de manière telle que le dispositif de déverrouillage (10) ne puisse pas être actionné de manière intempestive.
